Europäisches Patentamt

European Patent Office

Office européen des brevets

(19)

(11) Publication number: **0 040 601**
**B1**

(12) **EUROPEAN PATENT SPECIFICATION**

(45) Date of publication of patent specification: **14.03.84**

(51) Int. Cl.³: **C 12 Q 1/34, C 12 Q 1/56**
**//G01N33/68, G01N31/06**

(21) Application number: **80901644.7**

(22) Date of filing: **20.08.80**

(86) International application number:
**PCT/SE80/00214**

(87) International publication number:
**WO 81/00578 05.03.81 Gazette 81/6**

(54) METHOD FOR DETECTING PROTEOLYTIC ENZYMES IN BLOOD.

(30) Priority: **22.08.79 SE 7907013**

(43) Date of publication of application:
**02.12.81 Bulletin 81/48**

(45) Publication of the grant of the patent:
**14.03.84 Bulletin 84/11**

(84) Designated Contracting States:
**DE FR GB**

(56) References cited:
SE - A - 7 706 746
SE - A - 7 708 296
SE - B - 392 038
US - A - 3 920 625
US - A - 4 022 758
US - A - 4 106 990
US - A - 4 139 415

Diss. Abstr. 38 (1977), 178-79, Tzeng F.S.J

Thrombosis Research 11 (1977), 687-89, Pepper D.S., Prowse C. "Chromatography of human prothrombin complex on dextran sulphate agarose"

(73) Proprietor: **I.R.D. Biomaterial Aktiebolag**
**Box 20105**
**S-161 20 Bromma (SE)**

(72) Inventor: **LARSSON, Rolf Lennart**
**Rapsvägen 7**
**S-178 00 Ekerö (SE)**
Inventor: **OLSSON, Per Ingemar**
**Vikingagatan 10**
**S-113 43 Stockholm (SE)**

(74) Representative: **Burman, Tore et al,**
**Bergling & Sundbergh AB P.O. Box 7645**
**S-103 94 Stockholm (SE)**

(56) References cited:
**Thrombosis Research Vol 11, p 517-530, published 1977, Larsson R et al, "Determination of platelet adhesion to polyethylene and heparinized surfaces with the aid of bioluminiscence."**
**Thrombosis Research Vol 10, p 645-60, published 1977, Hatton M.W.C., Regeczi E., "The inactivation of thrombin and plasmin by antithrombin in the presence of sepharose-heparin."**
**Federation Proceedings Vol. 30, p 1494-1502, published 1971, Vroman L et al, "Interactions among human blood proteins at interfaces."**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 040 601

## Method for detecting proteolytic enzymes in blood

### Technical Field

The invention relates to a method of detecting proteolytic enzymes in blood by contacting the blood with an adsorbent to adsorb the enzymes on said adsorbent, and examining the presence of adsorbed enzymes. In the present specification and claims the term blood is used to indicate blood and fractions of blood, such as blood plasma and solutions of purified blood plasma proteins.

### Background Art

It is previously known that blood coagulation enzymes can be adsorbed in their active form on surfaces to which heparin or heparinoid substances have been bound. Such adsorbed enzymes, however, have been entirely inactivated upon contact with blood plasma or with enzyme inhibitors isolated from blood plasma. Thus, these known methods are not useful for detecting active enzymes in blood by means of a direct contact between the blood and an adsorbing surface, because the enzyme inhibitors of the blood inactivate the adsorbed enzymes.

In simplified terms, the equilibrium state of blood is regulated, in addition to cellular reactions, by the capacity of certain substances to inhibit the activity of said enzymes. From a diagnostic point of view it is desirable to be able to detect enzymes in blood and other body fluids, and preferably to measure the content of such enzymes. The inhibitor systems may be affected by the addition of e.g. heparin. Therefore, during an operation or in certain diseases, it is often important to be able to rapidly establish the presence of active enzymes in the blood, and, preferably, to determine the quantity of said active enzymes in the blood, in order to be able to determine the correct dose of heparin to be given to the patient. Earlier attempts to detect and measure free active enzymes in blood have been hampered by the fact that the inhibitor systems rapidly inactivated the enzymes during the collection of blood.

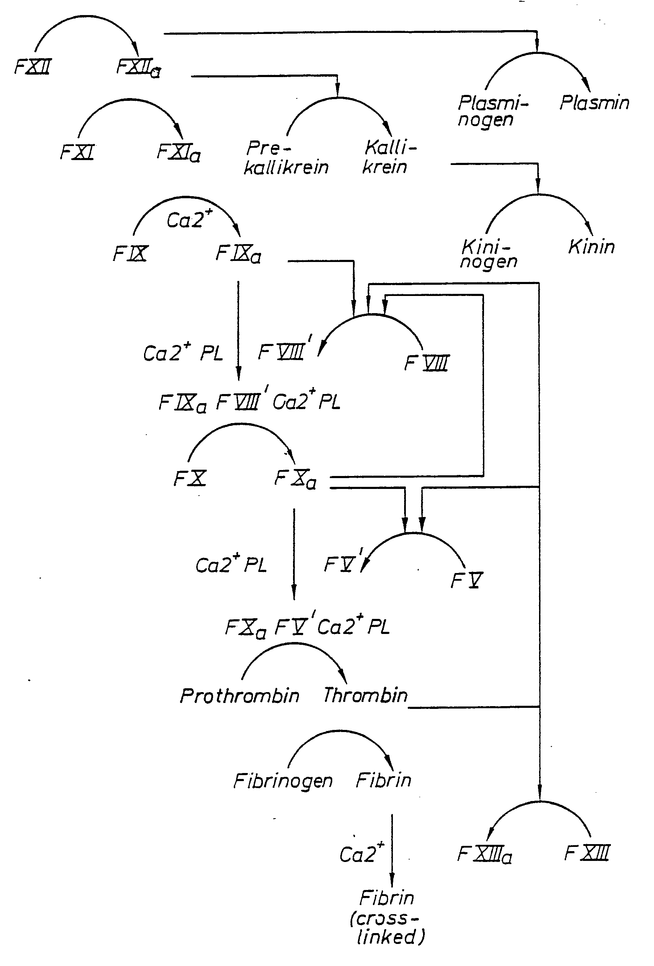

An enzyme usually occurs in blood in the form of an inactive protein. When being activated the protein is converted into an active enzyme by having a portion of the molecule split off. The enzymes are arranged in a cascade sequence, in which one enzyme activates another inactive protein so as to convert the latter into the next enzyme. The sequence for the blood coagulation system is illustrated in simplified form in the drawing. The enzymes are referred to as Factor I to Factor XIII, several enzymes also having other names. When reference is made to the active enzyme the index "a" is added, such as $X \rightarrow Xa$. Of particular interest is Factor Xa which is found in the intersection of the two pathways of activation referred to as the intrinsic pathway and the extrinsic pathway. Of particular interest is also thrombin (Factor IIa) which is the enzym that finally converts fibrinogen into insoluble fibrin. The drawing also discloses that the first enzyme of the sequence, Factor XIIa, also is involved in the activation of additional enzyme systems. One of these systems results in the formation of plasmin, which has a fibrinolytic acitivity. Another system results in the formation of kallikrein which is an intermediate product in the formation of kinin, which acts, i.a. upon the permeability of the blood vessels.

### The Invention

The object of the invention is to adsorb enzymes from blood under such circumstances that the enzymes have a remaining activity after having been adsorbed on the adsorbent. The invention aims at detecting proteolytic enzymes, such as serine enzymes, in various enzyme systems in blood, such as the blood coagulation system, the fibrinolytic system, the kallikrein system. The method of the invention is characterized by contacting the blood with an adsorbent consisting of a carrier (substratum surface) having sulphate groups fixed to its surface in such a high density that the enzymes are adsorbed and remain immobilized in their active forms even after exposure to the natural enzyme inhibitors existing in blood. The adsorbed enzymes can be detected by means of existing techniques while they are still present on the adsorbent, or after they have been dissolved by desorption. The invention is particularly devoted to determining the quantity of active enzymes in blood, which is possible with the enzymes referred to below.

We prefer to use an adsorbent consisting of a polymer material (plastic) which has been treated as described in the Swedish patent application No. 7706746-0. The surface of a polymer material consisting of polyethylene, polypropene, or polystyrene is treated with an oxidizing agent dissolved in concentrated sulphuric acid. The surface is now preferably rinsed with water, and is subsequently contacted with an aqueous solution of albumin. The oxidizing agent may be potassium permanganate, potassium dichromate, sodium chlorate, sodium perborate, hydrogen peroxide, or sodium peroxide. The content of oxidizing agent in the sulphuric acid is preferably from 1 millimole per liter up to a saturated solution. The polymer surface is preferabtly treated at room temperature for a time of a minute up to an hour. We prefer to treat polyethylene with potassium permanganate dissolved in concentrated sulphuric acid to a concentration of 12 millimoles per liter, for two minutes at room temperature. The surface thus treated appears to be somewhat unstable. Therefore, we prefer to coat the surface with a protein, thus increasing the stability. The protein is suitably albumin, preferably human albumin. The surface can be coated by being exposed to an aqueous solution of the protein, preferably containing at least 100 grams protein per liter. The surface is preferably treated at room temperature.

Other useful adsorbents are solid surfaces to which has been attached heparin or heparinoid

2

substances, such as chondroitin sulphate, heparan sulphate, dermatan sulphate, dextran sulphate, or other polysaccharides having a high content of sulphate groups in the molecule. When using heparin it is essential that the heparin is bound in a way which does not allow leakage of heparin from the surface during the initial phase of the blood contact, because desorbed heparin will occur in the layer of protein on the surface, thus inactivating the adsorbed enzyme. Therefore, such a surface is not useful according to this invention. A heparinized surface with no initial leakage of heparin which is useful according to the present invention can be prepared as disclosed in Larsson et al: Thrombosis Research, *15*, 157, 1979.

A carrier having an insoluble surface layer containing the sulphate-containing polysaccharides mentioned above can be produced by coating the surface with a complex compound of the polysaccharide and a cationic surfactant in the form of a primary amine. A useful coating process of this type has been described for heparin and a primary amine in the Swedish patents Nos. 306,597, 315,362, 365,710, 7,104,506-6, 7,503,240-9 and in the Swedish patent application No. 7,708,296-4. We prefer the coating method disclosed in said patent application, which comprises contacting the surface of the carrier with a colloidal solution of a complex compound of the polysaccharide and a cationic surfactant in the form of a primary amine. We prefer to use a colloidal solution in which the colloidal particles have a positive charge. Such a colloidal solution can be prepared by pouring a comparatively diluted aqueous solution of the polysaccharide into a comparatively concentrated aqueous solution of a primary amine, while stirring vigorously, care being taken that the mixture always contains primary amine in excess. In order to achieve an effective deposition of the positively charged colloidal particles on the surface of the carrier, we prefer to pre-treat said surface so as to introduce negative charges. This can be achieved by choosing a carrier of polymer material, and by sulphating the surface with the method described above with reference to the Swedish patent application No. 7,706,746-0.

When whole blood is used it is important that the adsorbent has such surface characteristics that the blood platelets (thrombocytes) are prevented from being activated and from adhering to the surface. The surface having a high density of surface-bound sulphate groups has the capacity of adsorbing a protein layer of such a selective composition that the platelets do not adhere or activate. The likely explanation is that fibrinogen does not adhere to the surface. The adsorbed protein layer has the capacity of binding proteolytic enzymes without said enzymes being inhibited, with the exception of a special heparin surface as mentioned above. The quantity of platelets can easily be assessed by means of bioluminiscense according to a method described in Larsson et al: Thrombosis Research, *11*, 517, 1977.

According to the invention the surface shall contain fixed sulphate groups in a high density. One way to establish experimentally that said conition is met, is as follows.

A number of test pieces, displaying the surface to be tested, are exposed to a solution of albumin for a few minutes. The test pieces are now exposed to a solution of albumin and thrombin for a few minutes, suitably 15 minutes. The solution should have a thrombin activity of approximately 20 units per milliliter, unit meaning a unit according to the NIH, i.e. the National Institute of Health. The test pieces are now divided into two groups. The pieces of the first group are incubated with physiological saline, and those of the second group with blood plasma having been defibrinogenated. A suitable incubation time is 10 minutes. The surface concentration of thrombin activity is now measured by incubating the test pieces with a substrate specific for Thrombin, viz. S-2238 from Kabi Diagnostica. The surface thus tested is useful according to the invention if the thrombin is not deactivated by the defibrinogenated plasma. The measured value for the surfaces incubated with defibrinogenated plasma shall not be lower than 50% of the measured value for the surfaces incubated with saline. The measured value for the surfaces incubated with saline should be at least $5 \times 10^{-4}$ units of absorbance per square centimeter and second, measured with the method described in Example 1. As an example of the required density of sulphate groups, a polyethylene surface treated with potassium permanganate dissolved in concentrated sulphuric acid should have a sulphate group density of at least $0.8 \times 10^{-5}$ millimoles per square centimeter.

The adsorbent may be shaped in any desired way. It may, for example, consist of a test tube in which the interior wall has been coated with a sulphated surface according to the invention. The test tube is filled with blood to be examined. After a certain time the test tube is emptied and rinsed with physiological saline. The test tube is now filled with a reagent specific to the enzyme to be detected, preferably a synthetic substrate. In another embodiment the adsorbent consists of a tube, both ends open, in which the interior wall has been coated with a sulphated surface. The tube is applied in an arteriovenous shunt in a patient, for example during an operation. The tube can be rapidly removed at any desired moment, and the presence of adsorbed enzymes can now be examined. Alternatively, the surface can be exposed to blood during the puncture of a vein. If it is required that the adsorbent should have a large surface, the adsorbent may have the form of small particles, such as spheres, having a sulphated surface according to the invention.

We prefer to detect adsorbed enzymes by means of commercially available synthetic substrates. For example, thrombin can be specifically detected by means of S-2238, Factor Xa by means of S-2222, Kallikrein by means of S-2302, and plasmin by means of S-2251, all these substrates originating from Kabi Diagnostica. It is also possible to detect adsorbed active enzymes in other ways. For example, the so-called contact product, consisting substantially of Factors XIIa, XIa and IXa, can be detected in this way.

A background will first be given. If citrated blood plasma is recalcified, the whole enzyme cascade will be put into operation, to proceed at a speed which depends on the degree of activation of Factors XII, XI and IX, said Factors being activated independent of calcium. The time period from the addition of calcium to a detectable formation of fibrin is called the recalcification time. An established way of producing contact activation is to incubate plasma in a glass tube, which results in a strong reduction of the recalcification time.

Test pieces having a high density of sulphate groups on their surfaces, and having preferably been pre-treated with albumin, are incubated for 10 minutes with normal plasma and with glass-activated plasma, and are subsequently rinsed with saline. All test pieces are now incubated with normal plasma, and the recalcification time is determined. It will be found that the recalcification time in those cases where glass-activated plasma was used during the first incubation is shorter than in those cases where normal plasma was used during the first incubation. This proves that the enzymes forming the contact product have been adsorbed on the sulphated surface.

Example 1

Polyethylene tubings having a length of 1 meter and an inner diameter of 1.8 millimeter were treated on the inside with concentrated sulphuric acid containing potassium permanganate in a concentration of 2 grams per liter, for 2 minutes at room temperature. After having been carefully rinsed with water the tubings were treated with a solution containing 4 per cent by weight albumin, for 5 minutes, and were subsequently carefully rinsed with physiological saline.

The tubings thus pre-treated were now tested as follows.

Four tubings were rotated for 15 minutes on a slanted turn-table. The tubings contained 1 milliliter physiological saline containing 4 per cent by weight albumin and 20 units per milliliter of thrombin. The tubings were subsequently carefully rinsed with saline. Two tubings were now rotated on the turntable for 5 minutes with defibrinogenated plasma, and were subsequently rinsed with saline. The presence of surface-bound thrombin activity was now investigated in this way. The tubings were divided into pieces having a length of 45 cm. These shorter tubings were incubated with 0.7 milliliter of a solution of substrate S-2238 which is specific to thrombin. The substrate had been dissolved in a Tris buffer having pH 8.4, according to the recommendations by the manufacturer. The substrate solution was sucked into the tubings by means of a reversible roller pump at a velocity of 2.5 centimeters per second, and the solution was pumped forward·and backward in the tubings by repeatedly reversing the pump for a total time of 80 seconds. Subsequently, the solution was pumped down into glacial acetic acid having a volume of 0.3 milliliter.

If thrombin is present the substrate will split off paranitroaniline. The quantity of paranitroaniline can easily be measured photometrically, and is proportional to the thrombin activity. The reaction is discontinued by changing the pH by means of the glacial acetic acid.

The absorbance of the solution was measured in a spectrophotometer using light having a wavelength of 405 nanometer and an optical pathway of 1 cm. The result was:

| | |
|---|---|
| Group 1 (rinsed with saline) | 1.100 |
| Group 2 (rinsed with saline and treated with defibrinogenated plasma) | 1.100 |

The values thus obtained can be compared with a standard curve obtained by incubating substrate solutions with various known amounts of thrombin. In this way the values given above can be estimated to correspond to a surface concentration of 0.6 unit of thrombin per square centimeter.

A similar experiment was made with polyethylene tubings which had not been pre-treated. The resulting absorbance was nil for both groups of tubings.

The experiments of this Example illustrate that thrombin is bound to the albumin layer and is withdrawn from being inactivated by the thrombin inhibitors in plasma.

Example 2

Polyethylene tubings having a length of 1 meter and an inner diameter of 1.8 millimeter were treated on the inside with concentrated sulphuric acid containing potassium permanganate in a concentration of 2 grams per liter, for 2 minutes at room temperature. After having been carefully rinsed the tubes were rotated with 1 milliliter of citrated plasma free from platelets for 60 minutes. Subsequently the tubes were rinsed carefully with physiological saline.

The tubings thus pre-treated were tested as described in Example 1. The resulting absorbance was:

# O 040 601

| | |
|---|---|
| Group 1 (saline) | 1.090 |
| Group 2 (defibrinogenated plasma) | 1.120 |

The Example illustrates that the sulphated surface, having been contacted with blood plasma, can bind thrombin according to the invention.

## Example 3

Polyethylene tubings having a length of 90 centimeters and an inner diameter of 3.7 millimeters were treated on the inside with concentrated sulphuric acid containing potassium permanganate in a concentration of 2 grams per liter, for 2 minutes at room temperature. After having been carefully rinsed the tubings were treated with a 4 per cent by weight solution of albumin and were subsequently rinsed with physiological saline.

The tubings thus pre-treated were tested in vivo in this way. The tubings were applied as arteriovenous shunts in dogs, viz. between Arteria and Vena femoralis. The tubings were compressed to produce a blood flow of approximately 40 milliliters per minute. After 20 minutes of exposure the tubings were removed, and were immediately rinsed with saline. A 30 centimeter length of each tube was now tested consecutively with four different enzyme substrates, in the way disclosed in Example 1, the buffer solutions being chosen as recommended by the manufacturer of the substrates. The substrates and the resulting absorbance values were:

| Substrate | Absorbance |
|---|---|
| S-2302 (kallikrein) | 0.342 |
| S-2251 (plasmin) | 0.325 |
| S-2222 (Factor Xa) | 0.239 |
| S-2238 (thrombin) | 0.388 |

This Example illustrates that said enzymes are adsorbed, without being inactivated, on a surface according to the invention upon contact with circulating blood in vivo.

## Example 4

Polyethylene tubings having a length of 1 meter and an inner diameter of 1.8 millimeter were treated on the inside with concentrated sulphuric acid containing potassium permanganate in a concentration of 2 grams per liter, for 2 minutes at room temperature. After having been carefully rinsed with water the tubings were treated with a solution containing 4 per cent by weight albumin, for 5 minutes, and were subsequently carefully rinsed with physiological saline.

One group of the tubings thus pre-treated were rotated with normal citrated plasma. A second group were rotated with citrated plasma which had been activated by rotation in a glass tube for 10 minutes at 37°C. All tubings were now carefully rinsed with physiological saline, and were rotated with normal citrated plasma for 10 minutes. The plasma samples from the last mentioned rotation, and control samples, were finally tested in a recalcification test. These results were obtained:

| Plasma sample: | Recalcification time, seconds: |
|---|---|
| Normal plasma, not rotated | 517 |
| Normal plasma, activated in a glass tube | 148 |
| Normal plasma, rotated in a sulphated tubing pre-rinsed with normal citrated plasma | 548 |
| Normal plasma, rotated in a sulphated tubing pre-rinsed with glass-activated plasma | 340 |

The Example illustrates that a surface having a layer of albumin according to the invention can adsorb the active enzymes Factors XIIa, XIa, IXa (the contact product) produced when citrated plasma is activated by contact with glass.

## Example 5

Polyethylene tubings having a length of 1 meter and an inner diameter of 1.8 millimeter were treated on the inside with concentrated sulphuric acid containing potassium permanganate in a

5

## 0 040 601

concentration of 2 grams per liter, for 2 minutes at room temperature, and were subsequently carefully rinsed with water.

The tubings thus pre-treated were tested in the way described in Example 1, with the difference that the thrombin was dissolved in saline free from albumin. The following absorbance values were found:

Group 1 (saline)                                                1.110

Group 2 (defibrinogenated plasma)                              1.080

The Example illustrates that the pure sulphated surfadce can bind thrombin.

### Claims

1. A method for detecting proteolytic enzymes in blood by contacting the blood with an adsorbent to adsorb the enzymes on said adsorbent, and examining the presence of adsorbed enzymes, characterized in contacting the blood with an adsorbent consisting of a carrier having sulphate groups fixed to its surface in such a high density that the enzymes are adsorbed and remain immobilized in their active form even after exposure to the natural enzyme inhibitors existing in blood.

2. A method as claimed in claim 1, characterized in examining the presence of serine enzymes adsorbed on the adsorbent.

3. A method as claimed in claim 1, characterized in examining the presence of blood coagulating enzymes on the adsorbent.

4. A method as claimed in claim 1, characterized in that the surface carrying fixed sulphate groups in a high density has been prepared by treating a polymer surface consisting of polyethylene, polypropene, or polystyrene with an oxidant dissolved in concentrated sulphuric acid.

### Revendications

1. Méthode pour déceler la présence dans le sang d'enzymes protéolytiques par mise en contact du sang avec un corps adsorbant pour y faire adsorber les enzymes, et examen de la présence ou non d'enzymes adsorbées, méthode caractérisée en ce que l'adsorbant est constitué par un support ayant des groupes sulfate liés à sa surface en densité telle que les enzymes y sont adsorbées et restent immobilisées sous leur forme active, même après une exposition à l'action des inhibiteurs d'enzymes naturels du sang.

2. Méthode selon la revendication 1, caractérisée en ce que l'on examine la présence sur l'adsorbant d'enzymes sériniques.

3. Méthode selon la revendication 1, caractérisée en ce que l'on examine la présence sur l'adsorbant d'enzymes de la coagulation du sang.

4. Méthode selon la revendication 1, 2 ou 3, caractérisée en ce que la surface portant une haute densité de groupes sulfate liés a été préparée par traitement, avec une solution d'un oxydant dans de l'acide sulfurique concentré, de la surface d'une matière polymère constituée par du polyéthylène, du polypropylène ou du polystyrène.

### Patentansprüche

1. Verfahren zum Bestimmen proteolytischer Enzyme im Blut durch Kontakt des Blutes mit einem Adsorbens, um die Enzyme auf dem Adsorbens zu adsorbieren, und Prüfung der Anwesenheit von adsorbierten Enzymen, dadurch gekennzeichnet, daß man das Blut mit einem Adsorbens in Berührung bringt, das aus einem Träger mit auf seiner Oberfläche in einer solch hohen Dichte fixierten Sulfatgruppen besteht, daß die Enzyme adsorbiert werden und in ihrer aktiven Form immobilisiert bleiben, selbst nachdem sie den im Blut vorliegenden natürlichen Enzyminhibitoren ausgesetzt wurden.

2. Verfahren nach Anspruch 1, dadurch gekennzeichnet, daß man die Gegenwart von auf dem Adsorbens adsorbierten Serinenzymen prüft.

3. Verfahren nach Anspruch 1, dadurch gekennzeichnet, daß man die Gegenwart von Blut koagulierenden Enzymen auf dem Adsorbens prüft.

4. Verfahren nach Anspruch 1, dadurch gekennzeichnet, daß die fixierte Sulfatgruppen in einer hohen Dichte tragende Oberfläche durch Behandlung einer Polymeroberfläche aus Polyäthylen, Polypropen oder Polystyrol mit einem in konzentrierter Schwefelsäure gelösten Oxidationsmittel hergestellt wurde.

FXII → FXIIₐ

FXIIₐ → FXI → FXIₐ

Pre-kallikrein → Kalli-krein

Plasmi-nogen → Plasmin

Kini-nogen → Kinin

$Ca2^+$

FIX → FIXₐ

$Ca2^+ PL$

$F VIII'$   F VIII

$FIX_a\ F VIII'\ Ca2^+ PL$

FX → FXₐ

$Ca2^+ PL$

$FV'$   FV

$FX_a\ FV'\ Ca2^+ PL$

Prothrombin → Thrombin

Fibrinogen → Fibrin

$Ca2^+$

Fibrin (cross-linked)

$F XIII_a$   F XIII

1